# EUROPÄISCHE PATENTSCHRIFT

(11) **EP 2 145 875 B1**
(45) Veröffentlichungstag und Bekanntmachung des Hinweises auf die Patenterteilung: **28.11.2012**
(21) Anmeldenummer: 08013045.3
(22) Anmeldetag: 19.07.2008
(51) Int. Cl.: C07C 211/63, C07C 303/24, C07C 305/10, C07C 305/04

(54) **Verfahren zur Herstellung von Onium-Verbindungen**
Method for producing onium compounds
Procédé destiné à la production de composés onium

(43) Veröffentlichungstag der Anmeldung: 20.01.2010
(73) Patentinhaber: Cognis IP Management GmbH, 40589 Düsseldorf (DE)
(72) Erfinder: Wiesenhoefer, Wolfgang, 40885 Ratingen (DE); Jepkens, Daniel, 41066 Mönchengladbach (DE)

(56) Entgegenhaltungen:
- WO-A-02/26701
- WO-A-03/074494
- WO-A-2006/033990
- WO-A-2006/050306
- WO-A-2006/063654
- US-A1- 2006 094 616
- XUEZHENG LIANG ET AL: "Synthesis of a Novel Strong Prönsted Acidic Ionic Liquid and its Catalytic Activities for the Oxathioacetalization" CATALYSIS LETTERS, Bd. 123, Nr. 3-4, 2008, Seiten 396-400, XP002510781

## Beschreibung

### Gebiet der Erfindung

Die Erfindung betrifft ein Verfahren zur Erstellung von speziellen QAV-Verbindungen.

### Stand der Technik

Unter ionischen Flüssigkelten versteht man im Allgemeinen organische Salze oder deren Gemische, deren Schmelzpunkt unterhalb von 100 °C liegt. Diese Salze bestehen typischerweise aus Anionen wie beispielsweise Halogeniden, Sulfaten, Ethersulfaten, Halogenostannaten, Halogenoaluminaten, Hexafluorophosphaten oder Tetrafluoroboraten kombiniert mit substituierten Ammonium-, Phosphonium-, Pyridinium-, Triazolium-, Pyrazolium- oder Imidazolium-Kationen. Solche Verbindungen, die als Anionen einen Sulfat- oder Ethersulfat-Baustein aufweisen, werden üblicherweise dadurch hergestellt, dass man ein entsprechendes Salz, das ein Halogenid-Anion aufweist, mit einem Natriumsalz eines Alkylsulfats oder einem Natriumsalz eines Alkylethersulfats umgesetzt wird. Bei dieser Reaktion entsteht eine große Menge an Kochsalz (NaCl) als Nebenprodukt, das durch aufwändlge Reinigungsoperationen in einem Folgeschritt abgetrennt werden muss.

WO 2006106354 A1 beschreibt ein Verfahren zur Herstellung von Onium Alkylsulfaten durch Umsetzung eines Onium Alkylhalogenids mit einem symmetrischen Dialkylsulfat bei Raumtemperatur.

WO 02/26701 A2 beschreibt onische Verbindungen mit einem Gefrierpunkt von bis zu 100 °C. Diese Verbindungen werden durch die Umsetzung spezieller Aminsalze (I) mit organischen Verbindungen (II), die eine Wasserstoffbrückenbindung mit Halogeniden ausbilden können, wie z.B. Harnstoff, wobei das molare Verhältnis von (I) und (11) im Bereich 1:1,5 bis 1:2,5 liegt, hergestellt.

US 2006/0094616 A1 beschreibt spezielle ionische Flüssigkeiten und Verfahren zu ihrer Herstellung. Insbesondere werden die ionischen Flüssigkeiten von Tensiden abgeleitet, etwa Betainen und Aminoxiden.

WO 03/074494 A1 beschreibt ionische Flüssigkelten, die ein Kation und ein Anion enthalten, wobei es sich bei dem Anion um Sulfat- oder Sulfonatgruppen enthaltende Verbindungen handelt. Die ionischen Flüssigkeiten sind halogenfrei.

WO 2006/033990 A2 betrifft die Verwendung ionischer Flüssigkeiten als koordinative Liganden für Organometall-Katalysatoren, die etwa bei der Umwandlung von Methan in Methanol zum Einsatz kommen.

### Beschreibung der Erfindung

Aufgabe der vorliegenden Erfindung war es, ein verbessertes Verfahren zur Herstellung von speziellen quaternären Ammoniumverbindungen (QAV) bereitzustellen, nämlich solchen, deren anionischer Teil durch einen Fettaikoholsulfat-, Fettaikoholethersulfat- oder Sulfonat-Baustein gekennzeichnet ist. Dabei sollten die Nachteile des bekannten Standes der Technik vermieden werden.

Gegenstand der vorliegenden Erfindung ist ein Verfahren zur Herstellung von ionischen Flüssigkeiten, deren anionischer Teil durch einen Fettalkoholsulfat-, Fettalkoholethersulfat- oder Sulfonat-Baustein gekennzeichnet ist, wobei man die entsprechende ionische Flüssigkeit, die denselben kationischen Baustein enthält, jedoch als anionischen Baustein ein Halogenid-Anion aufweist, mit einer Verbindung umsetzt, die ausgewählt ist aus der Gruppe (i) der sauren Halbester eines Fettalkoholsulfats oder eines Fettalkoholethersulfats und (ii) der Sulfonsäuren, und den bei der Reaktion entstehenden Halogenwasserstoff in Gasform aus dem Reaktionsgemisch entfernt.

Im Folgenden werden spezielle Ausführungsformen beschrieben, die sich hinsichtlich der beim erfindungsgemäßen Verfahren einzusetzenden ionischen Flüssigkeit - die allgemein durch die Formel [A]⁺ [Hal]⁻ beschrieben werden kann, wobei [A]⁺ ein kationischer Baustein und [Hal]⁻ ein Halogenid-Anion ist - in der Natur des kationischen Bausteins [A]⁺ unterscheiden. Um die hier in Frage kommenden Reste einerseits präzise zu definieren und andererseits umständliche und schwer lesbare sprachliche Formulierungen zu vermeiden, werden folgende Abkürzungen benutzt:
- Unter **U1** ist ein aliphatischer Rest zu verstehen, der insgesamt 1-24 C-Atome aufweist. Dieser Rest kann (a) gesättigt oder ein- bzw. mehrfach olefinisch ungesättigt sein; des weiteren kann er (b) linear oder ein- bzw. mehrfach verzweigt sein und schließlich kann er (c) ein oder mehrere alicyclische Elemente aufweisen.
- Unter **U2** ist ein aromatischer Rest zu verstehen, der insgesamt 5-18 C-Atome aufweist.
- Unter **U3** ist ein ein aliphatischer Rest zu verstehen, der insgesamt 1-12 C-Atome aufweist, wobei dieser Rest gesättigt oder ein bzw. mehrfach olefinisch ungesättigt sein kann, des weiteren kann er linear oder ein- bzw. mehrfach verzweigt sein; er kann ebenfalls ein oder mehrere alicyclische Elemente aufweisen. Zwingend ist jedoch die zusätzliche Maßgabe, dass dieser aliphatische Rest durch einen aromatischen Rest mit 5 bis 18 C-Atomen substituiert ist.
- Unter **U4** ist ein aromatischer Rest zu verstehen, der insgesamt 5-18 C-Atome aufweist, wobei zwingend die Maßgabe gilt, dass dieser aromatische Rest mit ein oder mehreren aliphatischen Gruppen mit jeweils 1 bis 12 C-Atomen substituiert ist, wobei diese aliphatischen Gruppen (a) gesättigt oder ein- bzw. mehrfach olefinisch ungesättigt sein können, (b) linear oder ein- bzw. mehrfach verzweigt sein können und (c) ein oder mehrere alicyclische Elemente aufweisen können.
- Unter **U5** ist ein Rest zu verstehen, der ausgewählt ist aus der Gruppe
   (a) -(X)ₙ-U1, wobei X Sauerstoff oder eine Gruppe NH, der Index n die Zahl 0 oder die Zahl 1 bedeutet und U1 die oben definierte Bedeutung hat,
   (b) -(X)ₙ-U2, wobei X Sauerstoff oder eine Gruppe NH, der Index n die Zahl 0 oder die Zahl 1 bedeutet und U2 die oben definierte Bedeutung hat,
   (c) -(X)ₙ-U3, wobei X Sauerstoff oder eine Gruppe NH, der Index n die Zahl 0 oder die Zahl 1 bedeutet und U3 die oben definierte Bedeutung hat

In einer Ausführungsform des erfindungsgemäßen Verfahrens setzt man eine Verbindung [A]⁺ [Hal]⁻,
wobei das Kation [A]⁺ ausgewählt ist aus der Gruppe der
- Quaternären Ammonium-Kationen der allgemeinen Formel [NR¹R²R³R⁴]⁺, wobei die Reste R¹ bis R⁴ unabhängig voneinander ausgewählt werden aus den Resten U1, U2, U3 und U4,
- Phosphonium-Kationen der allgemeinen Formel [PR⁵R⁶R⁷R⁸]⁺, wobei die Reste R⁵ bis R⁸ unabhängig voneinander ausgewählt werden aus den Resten U1, U2, U3 und U4,
- Imidazolium-Kationen der allgemeinen Formel 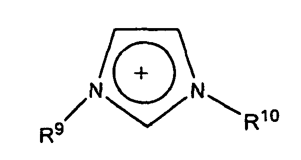 wobei die Reste R⁹ und R¹⁰ unabhängig voneinander ausgewählt werden aus den Resten Wasserstoff, U1, U2, U3, U4 und U5, mit der Maßgabe, dass nicht gleichzeitig beide Reste R⁹ und R¹⁰ Wasserstoff bedeuten, wobei gegebenenfalls der aromatische Ring des Imidazolium-Kations zusätzlich mit ein oder mehreren Resten substituiert ist, die ausgewählt sind aus den Resten U1, U2, U3, U4 und U5,
- Pyridinium-Kationen der allgemeinen Formel 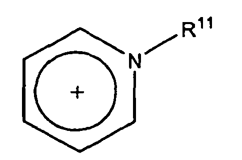 wobei der Rest R¹¹ ausgewählt wird aus den Resten Wasserstoff, U1, U2, U3, U4 und U5, wobei gegebenenfalls der aromatische Ring des Pyridinium-Kations zusätzlich mit ein oder mehreren Resten substituiert ist, die ausgewählt sind aus den Resten U1, U2, U3, U4 und U5,
- Pyrazolium-Kationen der allgemeinen Formel 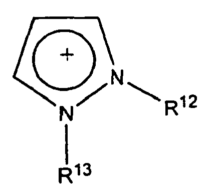 wobei die Reste R¹² und R¹³ ausgewählt wird aus den Resten Wasserstoff, U1, U2, U3, U4 und U5, mit der Maßgabe, dass nicht gleichzeitig beide Reste R⁹ und R¹⁰ Wasserstoff bedeuten, wobei gegebenenfalls der aromatische Ring des Pyrazoilum-Kations zusätzlich mit ein oder mehreren Resten substituiert ist, die ausgewählt sind aus den Resten U1, U2, U3, U4 und U5, und
- Triazolium-Kationen der allgemeinen Formel 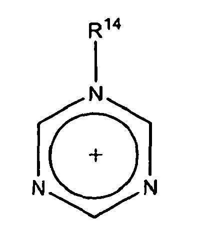 wobei der Rest R¹⁴ ausgewählt wird aus den Resten Wasserstoff, U1, U2, U3, U4 und U5, wobei gegebenenfalls der aromatische Ring des Triazolium-Kations zusätzlich mit ein oder mehreren Resten substituiert ist, die ausgewählt sind aus den Resten U1, U2, U3, U4 und U5,
wobei U1, U2, U3, U4 und U5 die oben genannte Bedeutung haben,
und der Rest [Hal]⁻ einen Halogenidrest bedeutet,
mit einer Verbindung der Formel R¹⁵-O-(CH₂-CH₂-O)ₓSO₃H oder einer Verbindung der allgemeinen Formel R¹⁶-SO₃H um, wobei die Reste R¹⁵ und R¹⁶ unabhängig voneinander einen Alkylrest mit 1 bis 22 C-Atomen oder einen Arylrest mit 6 bis 18 C-Atomen, der gegebenenfalls mit ein oder mehreren Alkylresten mit jeweils 1 bis 18 C-Atomen substituiert sein kann, bedeuten und der Index x Null oder eine Zahl von 1 bis 20 bedeutet.

Im Rahmen des erfindungsgemäßen Verfahrens setzt man besonders bevorzugt eine Verbindung vom Typ [NR¹R²R³R⁴]⁺ [Hal]⁻ ein, worin der Rest R¹ ein Benzylrest oder ein Alkylrest mit 1 bis 4 C-Atomen ist und die Reste R² bis R⁴ unabhängig voneinander Alkylreste mit 8 bis 10 C-Atomen sind, mit der Maßgabe, dass nicht alle Reste R² bis R⁴ identisch sind, und wobei der Rest [Hal]⁻ einen Chloridrest bedeutet.

Das erfindungsgemäße Verfahren ermöglicht es in eleganter Weise, nach dem Vermischen der beiden Reaktionskomponenten Halogenwasserstoff in Gasform aus dem Reaktionsgemisch zu entfernen, was in einfacher Weise z.B. durch Evakuieren geschehen kann. Das freigesetzte Gas (Halogenwasserstoff) kann durch beliebige aus dem Stand der Technik bekannte Methoden aufgefangen werden. Es kann dann als Wertstoff verwendet werden. Das Zielprodukt, nämlich die ionische Flüssigkeit, deren anionischer Teil durch einen Fettalkoholsulfat-, Fettalkoholethersulfat- oder Sulfonat-Baustein gekennzeichnet ist, kann unmittelbar und ohne weitere Reinigungsoperationen eingesetzt, d.h. unterschiedlichen Verwendungen zugeführt werden, z.B. als Lösungsmittel.

### Beispiele

### Eingesetzte Substanzen:

QAV: Quaternäre Ammoniumverbindung mit einem Wassergehalt unterhalb von 0,5 Gew.-%. Die Herstellung dieser Verbindung geschah wie folgt: 300g Aliquat 336 (Methyl-tri(octyl/decyl)-ammoniumchlorid; Handelsprodukt der Fa. Cognis) mit einem Wassergehalt von ca. 5 % Gew.-% wurden in einen Glaskolben gegeben und im Vakuum auf 50 °C erhitzt, bis der Wassergehalt unterhalb von 0,5 % Gew.-% betrug.

### Texapon N 70 - Halbester:

Schwefelsäurehalbester eines ethoxylierten C_{12/14}-Fettalkoholgemisches, erhältlich durch Sulfonierung von Dehydol LS2H (Anlagerungsprodukt von 1,85 mol Ethylenoxid an 1 mol eines Gemisches von C_{12/14}-Fettalkoholen - "Dehydol LS2H" ist ein Handelsprodukt der Firma Cognis) mit gasförmigem SO₃. Der Schwefelsäurehalbester wies eine Säurezahl von 153 auf.

### Sulfopon 12/18 - Halbester

Schwefelsäurehalbester eines C_{12/18}-Fettalkoholgemisches, erhältlich durch Sulfonierung von Lorol 12/18W (Gemisch von C_{12/18}-Fettalkoholen - "Lorol 12/18W" ist ein Handelsprodukt der Firma Cognis) mit gasförmigem SO₃. Der Schwefelsäurehalbester wies eine Säurezahl von 176 auf.

### Beispiel 1: Herstellung der ionischen Flüssigkeit IL1

140 g QAV wurden in einen Glaskolben mit Rührer gegeben. Anschließend wurden 114,46 g Texapon N 70 - Halbester zugefügt. Und die Mischung im Vakuum (Druck = 5 mbar.) bei 50 °C gerührt. Das Vakuum wurde auf 5 mbar reduziert und freigesetztes Gas (HCl) in einem Wäscher absorbiert. Diese Bedingungen wurden aufrechterhalten, bis die Gasbildung (HCl-Freisetzung) beendet war. Das Auswiegen des Rückstandes ergab 242,92 g, was auf einen vollständigen Umsatz hindeutet.

### Beispiel 2: Herstellung der ionischen Flüssigkeit IL2

110,9 g QAV wurden in einen Glaskolben mit Rührer gegeben. Anschließend wurden 82,6 g Sulfopon 12/18 - Halbester zugefügt. Und die Mischung im Vakuum (Druck = 5 mbar) bei 50 °C gerührt. Das Vakuum wurde auf 5 mbar reduziert und freigesetztes Gas (HCl) in einem Wäscher absorbiert. Diese Bedingungen wurden aufrechterhalten, bis die Gasbildung (HCl-Freisetzung) beendet war. Das Auswiegen des Rückstandes ergab 184,51 g, was auf einen vollständigen Umsatz hindeutet.

## Patentansprüche

1. Verfahren zur Herstellung von ionischen Flüssigkeiten, deren anionischer Teil durch einen Fettalkoholsulfat-, Fettalkoholethersulfat- oder Sulfonat-Baustein **gekennzeichnet ist, dadurch** gekennzeichnet, dass man die entsprechende ionische Flüssigkeit, die denselben kationischen Baustein enthält, jedoch als anionischen Baustein ein Halogenid-Anion aufweist, mit einer Verbindung umsetzt, die ausgewählt ist aus der Gruppe (i) der sauren Halbester eines Fettalkoholsulfats oder eines Fettalkoholethersulfats und (ii) der Sulfonsäuren, und den bei der Reaktion entstehenden Halogenwasserstoff in Gasform aus dem Reaktionsgemisch entfernt.

2. Verfahren nach Anspruch 1, wobei man eine ionische Flüssigkeit [A]⁺ [Hal]⁻, wobei das Kation [A]⁺ ausgewählt ist aus der Gruppe der
• Quaternären Ammonium-Kationen der allgemeinen Formel [NR¹R²R³R⁴]⁺, wobei die Reste R¹ bis R⁴ unabhängig voneinander ausgewählt werden aus den Resten U1, U2, U3 und U4,
• Phosphonium-Kationen der allgemeinen Formel [PR⁵R⁶R⁷R⁸]+, wobei die Reste R⁵ bis R⁸ unabhängig voneinander ausgewählt werden aus den Resten U1, U2, U3 und U4,
• Imidazolium-Kationen der allgemeinen Formel 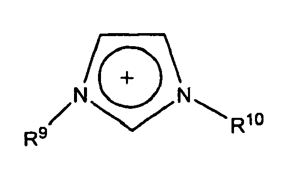 wobei die Reste R⁹ und R¹⁰ unabhängig voneinander ausgewählt werden aus den Resten Wasserstoff, U1, U2, U3, U4 und U5, mit der Maßgabe, dass nicht gleichzeitig beide Reste R⁹ und R¹⁰ Wasserstoff bedeuten, wobei gegebenenfalls der aromatische Ring des Imidazolium-Kations zusätzlich mit ein oder mehreren Resten substituiert ist, die ausgewählt sind aus den Resten U1, U2, U3, U4 und U5,
• Pyridinium-Kationen der allgemeinen Formel 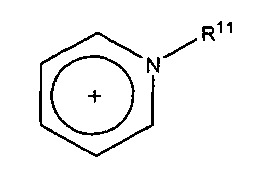 wobei der Rest R¹¹ ausgewählt wird aus den Resten Wasserstoff, U1, U2, U3, U4 und U5, wobei gegebenenfalls der aromatische Ring des Pyridinium-Kations zusätzlich mit ein oder mehreren Resten substituiert ist, die ausgewählt sind aus den Resten U1, U2, U3, U4 und U5,
• Pyrazolium-Kationen der allgemeinen Formel 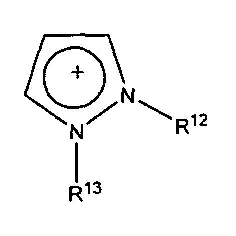 wobei die Reste R¹² und R¹³ ausgewählt wird aus den Resten Wasserstoff, U1, U2, U3, U4 und U5, mit der Maßgabe, dass nicht gleichzeitig beide Reste R⁹ und R¹⁰ Wasserstoff bedeuten, wobei gegebenenfalls der aromatische Ring des Pyrazoilum-Kations zusätzlich mit ein oder mehreren Resten substituiert ist, die ausgewählt sind aus den Resten U1, U2, U3, U4 und U5, und
• Triazolium-Kationen der allgemeinen Formel 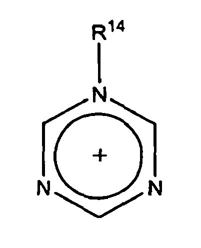 wobei der Rest R¹⁴ ausgewählt wird aus den Resten Wasserstoff, U1, U2, U3, U4 und U5, wobei gegebenenfalls der aromatische Ring des Triazolium-Kations zusätzlich mit ein oder mehreren Resten substituiert ist, die ausgewählt sind aus den Resten U1, U2, U3, U4 und U5,
wobei U1, U2, U3, U4 und U5 jeweils die folgende Bedeutung haben:
• unter U1 ist ein aliphatischer Rest zu verstehen, der insgesamt 1-24 C-Atome aufweist. Dieser Rest kann (a) gesättigt oder ein- bzw. mehrfach olefinisch ungesättigt sein; des weiteren kann er (b) linear oder ein- bzw. mehrfach verzweigt sein und schließlich kann er (c) ein oder mehrere alicyclische Elemente aufweisen.
• unter U2 ist ein aromatischer Rest zu verstehen, der insgesamt 5-18 C-Atome aufweist.
• unter U3 ist ein aliphatischer Rest zu verstehen, der insgesamt 1-12 C-Atome aufweist, wobei dieser Rest gesättigt oder ein bzw. mehrfach olefinisch ungesättigt sein kann, des weiteren kann er linear oder ein- bzw. mehrfach verzweigt sein; er kann ebenfalls ein oder mehrere alicyclische Elemente aufweisen. Zwingend ist jedoch die zusätzliche Maßgabe, dass dieser aliphatische Rest durch einen aromatischen Rest mit 5 bis 18 C-Atomen substituiert ist.
• unter U4 ist ein aromatischer Rest zu verstehen, der insgesamt 5-18 C-Atome aufweist, wobei zwingend die Maßgabe gilt, dass dieser aromatische Rest mit ein oder mehreren aliphatischen Gruppen mit jeweils 1 bis 12 C-Atomen substituiert ist, wobei diese aliphatischen Gruppen (a) gesättigt oder ein- bzw. mehrfach olefinisch ungesättigt sein können, (b) linear oder ein- bzw. mehrfach verzweigt sein können und (c) ein oder mehrere alicyclische Elemente aufweisen können.
• unter U5 ist ein Rest zu verstehen, der ausgewählt ist aus der Gruppe
-(X)ₙ-U1, wobei X Sauerstoff oder eine Gruppe NH, der Index n die Zahl 0 oder die Zahl 1 bedeutet und U1 die oben definierte Bedeutung hat,
-(X)ₙ-U2, wobei X Sauerstoff oder eine Gruppe NH, der Index n die Zahl 0 oder die Zahl 1 bedeutet und U2 die oben definierte Bedeutung hat,
-(X)ₙ-U3, wobei X Sauerstoff oder eine Gruppe NH, der Index n die Zahl 0 oder die Zahl 1 bedeutet und U3 die oben definierte Bedeutung hat
und der Rest [Hal]⁻ einen Halogenidrest bedeutet,
mit einer Verbindung der Formel R¹⁵-O-(CH₂-CH₂-O)ₓSO₃H oder einer Verbindung der allgemeinen Formel R¹⁶-SO₃H umsetzt, wobei die Reste R¹⁵ und R¹⁶ unabhängig voneinander einen Alkylrest mit 1 bis 22 C-Atomen oder einen Arylrest mit 6 bis 18 C-Atomen, der gegebenenfalls mit ein oder mehreren Alkylresten mit jeweils 1 bis 18 C-Atomen substituiert sein kann, bedeuten und der Index x Null oder eine Zahl von 1 bis 20 bedeutet.

## Claims

1. Process for preparing ionic liquids whose anionic part is **characterized by** a fatty alcohol sulphate, fatty alcohol ether sulphate or sulphonate building block, **characterized in that** the corresponding ionic liquid which contains the same cationic building block but has a halide anion as anionic building block is reacted with a compound selected from the group consisting of (i) the acid monoesters of a fatty alcohol sulphate or a fatty alcohol ether sulphate and (ii) sulphonic acids and the hydrogen halide formed in the reaction is removed in gaseous form from the reaction mixture.

2. Process according to Claim 1, wherein an ionic liquid [A]⁺ + [Hal]⁻, where the cation [A]⁺ is selected from the group consisting of
• quaternary ammonium cations of the general formula [NR¹R²R³R⁴]+, where the radicals R¹ to R⁴ are selected independently from among the radicals U1, U2, U3 and U4,
• phosphonium cations of the general formula [PR⁵R⁶R⁷R⁸]+, where the radicals R⁵ to R⁸ are selected independently from among the radicals U1, U2, U3 and U4,
• imidazolium cations of the general formula 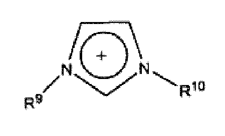 where the radicals R⁹ and R¹⁰ are selected independently from among the radicals hydrogen, U1, U2, U3, U4 and U5, with the proviso that the two radicals R⁹ and R¹⁰ are not simultaneously hydrogen, where the aromatic ring of the imidazolium cation is optionally additionally substituted by one or more radicals selected from among the radicals U1, U2, U3, U4 and U5,
• pyridinium cations of the general formula 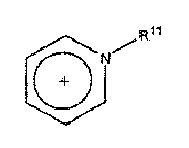 where the radical R¹¹ is selected from among the radicals hydrogen, U1, U2, U3, U4 and U5, where the aromatic ring of the pyridinium cation is optionally additionally substituted by one or more radicals selected from among the radicals U1, U2, U3, U4 and U5,
• pyrazolium cations of the general formula 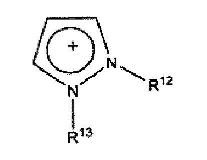 where the radicals R¹² and R¹³ are selected from among the radicals hydrogen, U1, U2, U3, U4 and U5, with the proviso that the two radicals R¹² and R¹³ are not simultaneously hydrogen, where the aromatic ring of the pyrazolium cation is optionally additionally substituted by one or more radicals selected from among the radicals U1, U2, U3, U4 and U5 and
• triazolium cations of the general formula 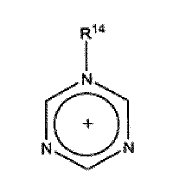 where the radical R¹⁴ is selected from among the radicals hydrogen, U1, U2, U3, U4 and U5, where the aromatic ring of the triazolium cation is optionally additionally substituted by one or more radicals selected from among the radicals U1, U2, U3, U4 and U5,
where U1, U2, U3, U4 and U5 have the following meanings:
• U1 is an aliphatic radical which has a total of 1-24 carbon atoms; this radical can be (a) saturated or olefinically monounsaturated or polyunsaturated; furthermore, it can be (b) linear or singly or multiply branched and finally it can have (c) one or more alicyclic elements;
• U2 is an aromatic radical having a total of 5-18 carbon atoms;
• U3 is an aliphatic radical having a total of 1-12 carbon atoms, where this radical can be saturated or olefinically monounsaturated or polyunsaturated and it can also be linear or singly or multiply branched; it can likewise have one or more alicyclic elements; however, the additional proviso that this aliphatic radical is substituted by an aromatic radical having from 5 to 18 carbon atoms is a necessary condition;
• U4 is an aromatic radical having a total of 5-18 carbon atoms, where the proviso that this aromatic radical is substituted by one or more aliphatic groups each having from 1 to 12 carbon atoms, where these aliphatic groups (a) can be saturated or olefinically monounsaturated or polyunsaturated, (b) can be linear or singly or multiply branched and (c) can have one or more alicyclic elements, is a necessary condition;
• U5 is a radical selected from the group consisting of
-(X)ₙ-U1, where X is oxygen or an NH group, the index n is 0 or 1 and U1 is as defined above,
-(X)ₙ-U2, where X is oxygen or an NH group, the index n is 0 or 1 and U2 is as defined above,
-(X)ₙ-U3, where X is oxygen or an NH group, the index n is 0 or 1 and U3 is as defined above,
and the radical [Hal]⁻ is a halide radical,
is reacted with a compound of the formula R¹⁵-O- (CH₂-CH₂-O)_{X}SO₃H or a compound of the general formula R¹⁶-SO₃H, where the radicals R¹⁵ and R¹⁶ are each, independently of one another, an alkyl radical having from 1 to 22 carbon atoms or an aryl radical which has from 6 to 18 carbon atoms and can optionally be substituted by one or more alkyl radicals each having from 1 to 18 carbon atoms and the index x is zero or from 1 to 20.

## Revendications

1. Procédé de fabrication de liquides ioniques, dont la partie anionique est **caractérisée par** un constituant sulfate d'alcool gras, éther-sulfate d'alcool gras ou sulfonate, **caractérisé en ce que** le liquide ionique correspondant, qui contient le même constituant cationique, mais qui comprend toutefois un anion halogénure en tant que constituant anionique, est mis en réaction avec un composé choisi dans le groupe (i) des semi-esters acides d'un sulfate d'alcool gras ou d'un éther-sulfate d'alcool gras et (ii) des acides sulfoniques, et l'halogénure d'hydrogène formé lors de la réaction est éliminé sous forme gazeuse du mélange réactionnel.

2. Procédé selon la revendication 1, dans lequel un liquide ionique [A]⁺[Hal]⁻, le cation [A]⁺ étant choisi dans le groupe des
- cations ammonium quaternaire de formule générale [NR¹R²R³R⁴]⁺, les radicaux R¹ à R⁴ étant choisis indépendamment les uns des autres parmi les radicaux U1, U2, U3 et U4,
- les cations phosphonium de formule générale [PR⁵R⁶R⁷R⁸]⁺, les radicaux R⁵ à R⁸ étant choisis indépendamment les uns des autres parmi les radicaux U1, U2, U3 et U4,
- les cations imidazolium de formule générale 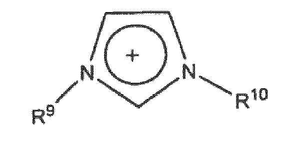 les radicaux R⁹ et R¹⁰ étant choisis indépendamment l'un de l'autre parmi les radicaux hydrogène, U1, U2, U3, U4 et U5, à condition que les deux radicaux R⁹ et R¹⁰ ne signifient pas simultanément l'hydrogène, le cycle aromatique du cation imidazolium étant éventuellement également substitué avec un ou plusieurs radicaux choisis parmi les radicaux U1, U2, U3, U4 et U5,
- les cations pyridinium de formule générale 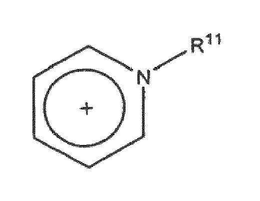 le radical R¹¹ étant choisi parmi les radicaux hydrogène, U1, U2, U3, U4 et U5, le cycle aromatique du cation pyridinium étant éventuellement également substitué avec un ou plusieurs radicaux choisis parmi les radicaux U1, U2, U3, U4 et U5,
- les cations pyrazolium de formule générale 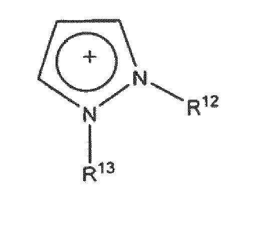 les radicaux R¹² et R¹³ étant choisis parmi les radicaux hydrogène, U1, U2, U3, U4 et U5, à condition que les deux radicaux R¹² et R¹³ ne signifient pas simultanément l'hydrogène, le cycle aromatique du cation pyrazolium étant éventuellement également substitué avec un ou plusieurs radicaux choisis parmi les radicaux U1, U2, U3, U4 et U5, et
- les cations triazolium de formule générale 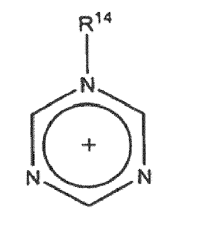 le radical R¹⁴ étant choisi parmi les radicaux hydrogène, U1, U2, U3, U4 et U5, le cycle aromatique du cation triazolium étant éventuellement également substitué avec un ou plusieurs radicaux choisis parmi les radicaux U1, U2, U3, U4 et U5,
U1, U2, U3, U4 et U5 ayant chacun la signification suivante :
- U1 se rapporte à un radical aliphatique qui comprend au total 1 à 24 atomes C. Ce radical peut (a) être saturé ou oléfiniquement insaturé une ou plusieurs fois ; il peut également (b) être linéaire ou ramifié une ou plusieurs fois, et enfin il peut (c) comprendre un ou plusieurs éléments alicycliques.
- U2 se rapporte à un radical aromatique qui comprend au total 5 à 18 atomes C.
- U3 se rapporte à un radical aliphatique qui comprend au total 1 à 12 atomes C, ce radical pouvant être saturé ou oléfiniquement insaturé une ou plusieurs fois, pouvant également être linéaire ou ramifié une ou plusieurs fois ; et pouvant également comprendre un ou plusieurs éléments alicycliques. Une condition supplémentaire obligatoire est toutefois que ce radical aliphatique soit substitué par un radical aromatique de 5 à 18 atomes C.
- U4 se rapporte à un radical aromatique, qui comprend au total 5 à 18 atomes C, une condition obligatoire étant que ce radical aromatique soit substitué avec un ou plusieurs groupes aliphatiques contenant chacun 1 à 12 atomes C, ces groupes aliphatiques (a) pouvant être saturés ou oléfiniquement insaturés une ou plusieurs fois, (b) pouvant être linéaires ou ramifiés une ou plusieurs fois et (c) pouvant comprendre un ou plusieurs éléments alicycliques.
- U5 se rapporte à un radical choisi dans le groupe
-(X)ₙ-U1, X signifiant oxygène ou un groupe NH, l'indice n signifiant le nombre 0 ou le nombre 1 et U1 ayant la signification définie précédemment,
-(X)ₙ-U2, X signifiant oxygène ou un groupe NH, l'indice n signifiant le nombre 0 ou le nombre 1 et U2 ayant la signification définie précédemment,
-(X)ₙ-U3, X signifiant oxygène ou un groupe NH, l'indice n signifiant le nombre 0 ou le nombre 1 et U3 ayant la signification définie précédemment,
et le radical [Hal]⁻ signifiant un radical halogénure, est mis en réaction avec un composé de formule R¹⁵-O-(CH₂-CH₂-O)ₓSO₃H ou un composé de formule générale R¹⁶-SO₃H, les radicaux R¹⁵ et R¹⁶ signifiant indépendamment l'un de l'autre un radical alkyle de 1 à 22 atomes C ou un radical aryle de 6 à 18 atomes C, qui peut éventuellement être substitué avec un ou plusieurs radicaux alkyle contenant chacun 1 à 18 atomes C, et l'indice x signifiant zéro ou un nombre de 1 à 20.
